# EUROPEAN PATENT SPECIFICATION

(11) **EP 1 589 945 B1**
(45) Date of publication and mention of the grant of the patent: **18.05.2011**
(21) Application number: 04704643.8
(22) Date of filing: 23.01.2004
(51) Int. Cl.: A61K 9/06, A61K 8/02, A61K 8/368, A61Q 19/00, B65D 81/32, B05B 11/00

(54) **TOPICAL PHARMACEUTICAL AND/OR COSMETIC DISPENSE SYSTEMS**
TOPISCHE PHARMAZEUTISCHE UND/ODER COSMETISCHE DOSIERUNGSSYSTEME
SYSTEME POUR DISTRIBUER DES PRODUITS PHARMACEUTIQUES ET/OU COSMETIQUES

(30) Priority: 23.01.2003 GB 0301577
(43) Date of publication of application: 02.11.2005
(73) Proprietor: EDKO Pazarlama Tanitim Ticaret Limited Sirketi, Sisli 34398 Istanbul (TR)
(72) Inventor: EMBIL, Koral, Eski Buyukdere Caddesi, 34398 Istanbul (Maslak) (TR); NACHT, Sergio, Las Vegas, NV 89135 (US)
(74) Representative: Golding, Louise Ann
(86) International application number: PCT/GB2004/000288
(87) International publication number: WO 2004/064803

(56) References cited:
- WO-A-01/91726
- WO-A-93/15726
- CA-A- 2 015 111
- US-A- 5 879 716
- US-A- 5 955 109
- US-A1- 2003 008 851

## Description

This invention relates to systems for dispensing topical pharmaceutical and/or cosmetic formulations, in particular pharmaceutical formulations for the treatment of dermatological conditions such as acne, rosacea, melasma (chloasma), alopecia, fungal infections, bacterial infections, viral infections, psoriasis, eczema and the like, and cosmetic formulations for treating conditions such as premature aging (e.g. wrinkling) of the skin. Such systems may also be used for dispensing formulations for treating conditions which affect the mucosal membranes, e.g. the membranes of the genital area, such as vulvitis, balanitis, etc., and haemorrhoidal conditions.

It has long been the case that pharmaceutical formulations, typically in the form of creams or gels suitable for topical administration, have been used to combat skin disorders which are caused by the inflammation of sebaceous glands and/or skin follicles and which may result in conditions such as acne and rosacea. Acne is one of the most common skin disorders which is particularly common during puberty but which may persist for many years. The main factors which contribute to the pathogenesis of acne are comedogenesis, sebum production, inflammation of the sebaceous glands and the presence of the bacteria *Propionibacterium acnes* (*P. acnes*).

Many previous treatments for such skin disorders can be found in the literature and often comprise topically administrable organic peroxides, retinoids and/or antibiotics. Thus, for example, formulations for the treatment of skin disorders comprising benzoyl peroxide are described in US-A-3535422, US-A-4056611, US-A-4318907, US-A-4923900, US-A-4387107 and US-A-4228163.

Other patents have disclosed formulations comprising an antibiotic for the treatment of skin disorders such as acne and rosacea. US-A-3969516, for example, describes the use of the topical antibiotic clindamycin for the treatment of acne. Other antibiotics which have been used in the treatment of skin disorders include erythromycin and tetracyclines.

Still further patents disclose the use of combination preparations which comprise actives having complementary but distinct mechanisms of action, for example formulations comprising a combination of an organic peroxide and an antibiotic for the treatment of skin disorders. GB-A-2088717, US-A-4411893, US-A-4692329 and GB-A-1594314, for example, describe combinations of the antibiotic erythromycin with various organic peroxides as formulations for the treatment of acne or other skin disorders. US-A-4607101 discloses a formulation for the treatment of acne vulgaris comprising a carbamide peroxide in combination with a topical antibiotic.

It has been found that combinations of an antibiotic with an organic peroxide may be more effective in the treatment of skin disorders than either the antibiotic or the peroxide alone - see, for example, US-A-4497794.

Benzoyl peroxide is an antibacterial, keratolytic and desquamating agent. Like other organic peroxides, it exerts an antibacterial effect via its strong oxidising properties. This chemical property of peroxides, however, can reduce the stability of such peroxide-containing two ingredient formulations, since oxidative interaction with the second (e.g. antibiotic) ingredient may lead to loss of potency of both active ingredients.

Benzamycin^{®} (Dermik Lab.) is a topical gel for the treatment of acne comprising a combination of 3% erythromycin, as a topical antibiotic, and 5% benzoyl peroxide, as an antibacterial, keratolytic and desquamating agent. This combination, however, is unstable at room temperature for the above reason, so that Benzamycin^{®} rapidly loses its pharmaceutical effectiveness if stored at ambient temperature.

In order to prolong the pharmaceutical effectiveness of Benzamycin^{®} it must be formulated by a pharmacist as and when required, thereafter being stored under refrigeration. However, this limits the product to being sold in a pharmacy and may also lead to variation in the final composition. Thus the pharmacist is required firstly to dissolve the erythromycin in an alcohol and then to add the resulting solution to a gel containing the benzoyl peroxide, thereafter stirring the mixture until it is homogeneous in appearance. Accordingly, variations in the alcohol used for this purpose may lead to variability in the product; if the mixing is not complete, partially dissolved or undissolved drug aggregates may remain, which may lead the product to feel gritty on application and, more importantly, to a partial loss of efficacy. Moreover, it may be impractical for a patient to store Benzamycin^{®} in a refrigerator (for example, when the patient is travelling or does not have access to a refrigerator); the need for refrigeration may also reduce patient compliance, since the application of a cold topical formulation may well be unpleasant.

Various attempts have been made to overcome the instability of formulations such as Benzamycin^{®}. US-A-5446028, US-A-5767098 and US-A-6013637, for instance, disclose formulations further comprising a stabilising agent such as dioctyl sodium sulfosuccinate. US-A-5466446 discloses a method for preparing a reportedly stable formulation comprising clindamycin and benzoyl peroxide by controlling the ratio of each active ingredient. The proprietor of this patent markets a product under the name Clindoxyl^{®} Gel which contains benzoyl peroxide and clindamycin in the ratio of 5:1 and which has a shelf-life of 60 days at room temperature. The product is, however, required to be kept refrigerated prior to being dispensed, which is inconvenient as well as impractical.

US-A-6117843 discloses compositions wherein the ratio of organic peroxide to antibiotic is a factor in achieving stability in the final composition. According to this patent a composition comprising benzoyl peroxide and clindamycin is prepared by a pharmacist by mixing an aqueous solution of clindamycin, typically having a pH of 5 to 6.5, with an aqueous suspension of benzoyl peroxide, typically having a pH of about 4 to 5. The benzoyl peroxide suspension preferably also contains a gelling agent with a pH-dependent viscosity, so that the viscosity of the product is increased when the two components are mixed. A gel composition is therefore obtained which is reported to be stable for around three months at room temperature.

Benzaclin^{®} is the only FDA-approved combination of 1% clindamycin phosphate and 5% benzoyl peroxide gel. Although this can be stored at room temperature (up to 25°C), this is only stable for about two months.

Other attempts to overcome the stability problems of combined topical antibacterial/antibiotic formulations include presenting the components as separate formulations which may be mixed in a controlled ratio on demand, e.g. immediately prior to, during or following application to the skin. For example, US-A-6462025 discloses that separate antibiotic and benzoyl peroxide compositions may be packaged within and dispensed from a common dispenser such as a dual chamber storage device. In this way the active ingredients are kept apart during storage, being dispensed and mixed as required immediately prior to application to the skin; long shelf life may thereby be achievable.

However, US-A-6462025 requires at least the antibiotic to be formulated in a "substantially anhydrous" composition comprising a polar solvent such as a polyol and a thickening agent which is a (meth)acrylic acid polymer or a poly(meth)acrylamide. Other than water of hydration which may be present in the various components used to formulate the composition, no free water is added to the composition such that its water content is less than 5% by weight. It is suggested that the benzoyl peroxide may also be presented in a "substantially anhydrous" polar solvent- and thickening agent-containing composition; for reasons of "cosmetic elegance" this should preferably have a viscosity differing by no more than 25% from that of the antibiotic composition.

Such thickened substantially anhydrous gels and like compositions are not, however, ideal medicaments for the treatment of skin disorders, since their use may lead to blockage of pores and/or the bases of hair follicles in a patient's skin, thereby potentially exacerbating conditions such as acne.

The present invention is based on the finding that dual (or multi) formulation topical pharmaceutical products having significantly improved effects in the treatment of dermatological conditions such as acne may be obtained by using separate water-based (i.e. essentially "non-anhydrous") formulations for each active ingredient. The improvement is achieved by incorporating at least one of the active ingredients into a polymeric delivery system capable of delayed release of the active, e.g. a polymeric system comprising porous polymer particles, and by using water-based (in particular, aqueous) carrier bases with substantially the same lipophilicity in each of the formulations.

Thus it has been found that the use of water-based or aqueous carriers optimises performance of polymeric delivery systems, both by ensuring slow continuous release of active ingredient from within the lipophilic environment of the delivery system and by enhancing the ability of the polymeric delivery system to absorb excess oil and sebum from the skin. This latter property is highly beneficial given that excess sebum content of the skin leads to blockage of pores and hair follicles, which in turn may lead to inflammation of the skin and development of acne.

The requirement for the formulations to comprise carrier bases with substantially the same lipophilicity is also an important feature of the invention which may facilitate particularly ready, uniform and thermodynamically favourable mixing of the formulations. More importantly, it ensures consistent release of active ingredient from the polymeric delivery system or systems. The release properties of such systems are dependent on the physical properties of the carrier in which they are dispersed, including pH and viscosity; thus, the degree of lipophilicity is particularly important since it affects the partition coefficient of active ingredient between the polymer particles of the delivery system and the carrier and thus controls the rate of release of active ingredient from the particles into the carrier and thus to the skin. By using carriers with substantially identical lipophilicity the products may be designed to ensure that a desired rate of release from the polymeric delivery system is consistently achieved after the formulations have been mixed and applied to the skin.

Excellent storage stability (e.g. six months or more, preferably in excess of 12, 18 or even 24 months) may be achieved by presenting the active ingredients in separate formulations which may be mixed immediately prior to, during or following application to the skin of a patient. In the case of systems involving oxidising antibacterials such as benzoyl peroxide and antibiotics such as clindamycin, highly efficacious formulations with a storage life in excess of two years at ambient temperature may be prepared in this way. The principle may also be applied to any other topical pharmaceutical and/or cosmetic formulations involving components which may potentially be mutually incompatible, e.g. as a result of chemical interaction. It is also generally applicable to dual and multi formulation topical pharmaceuticals and cosmetics in which at least one of the formulations involves a polymeric delivery system.

Thus according to one aspect of the present invention there is provided a pharmaceutical and/or cosmetic product comprising first and second active ingredient-containing formulations for topical administration to a patient, wherein said product includes storage means whereby said formulations are maintained separately prior to dispense, together with dispense means which permit said formulations to be dispensed from said storage means; characterised in that (i) an active ingredient in at least one of said formulations is contained within a polymeric delivery system and (ii) both of said formulations comprise water-based (e.g. aqueous) carrier bases having substantially the same lipophilicity, i.e. in which the partition coefficient of said active ingredient between said polymer particles and each of the carrier bases varies by no more than 10%, wherein said first and second formulations have viscosities which vary by no more than 10%.

It will be appreciated that the invention also embraces products comprising more than two formulations as defined above provided that these are each separately maintained in appropriate storage means prior to dispense, that all comprise water-based (for example, aqueous) carrier bases having substantially the same lipophilicity, and that at least one active ingredient is contained within a polymeric delivery system.

The storage means within products of the invention may, for example, comprise separate chambers or compartments of a dual- or multi- chamber or compartment dispense device, for example side-by-side collapsible tubes, syringe barrels or other forms of container with appropriate dispense valves, pistons, plungers or the like. A product comprising two (or more) separate chambers (e.g. made from polypropylene) provided with two (or more) fixed dosing units in a single cap is particularly suitable. Alternatively the storage means may take the form of a pouch containing a single unit dose of each formulation; such unit dose pouches may, for example, be made of composite materials such as a metal (e.g. aluminium) foil having a plastics material (e.g. polyethylene or polyvinyl chloride) inner lining, with the pouch having separate parts for each formulation. Examples of suitable pouches from which the formulations may be dispensed include those described in US-A-6007264 and WO 01/91726.

The dispense means are preferably such that the formulations are dispensable in a controllable (e.g. predetermined) ratio, for example in equal amounts or in other relative amounts as determined to optimise the efficacy of the combined formulation after mixing. In this way variations in the composition of the mixed product may be substantially reduced or eliminated. Thus, for example, side-by-side tube containers may be progressively emptied by turning a common winding key adapted to engage and roll up their distal ends, or the plungers of side-by-side syringes may be mutually linked together. It will be appreciated that the respective cross-sectional areas of such containers may be selected to ensure that the formulations are dispensed in the desired ratio for a given movement of a linked dispensing actuator.

In the case of unit dose pouches, the ratio of the formulations is predetermined at the filling stage, so that the dispense means need comprise no more than corners or edges which may be cut off or torn off to permit the formulations to be squeezed out.

In general it is convenient to dispense the formulations of a product of the invention in equal amounts, for example each in volumes in the range 1-5 ml, advantageously 1.5-3.5 ml, preferably 2-3 ml.

In a preferred product according to the invention, first and second formulations are respectively presented in the chambers of a dual chamber dispense system of the type described in EP-A-0644129 and US-A-5356040.

Such a system has two side-by-side chambers, each equipped with a dispense valve; these are operated by adjacent actuators so as to dispense the formulations either simultaneously or separately as desired. Suitable dispense systems, e.g. having chambers which are each capable of holding about 15 ml of formulation, are available from Maplast S.r.l., Via Pasublo 3, Tradate 21049 VA, Italy. The respective dimensions of the dispense means may be chosen to provide dispense of the respective formulations in a predetermined ratio.

The product may include mixing means such that the formulations are admixed during dispense. Thus, for example, the points of dispense for each of the storage means may be connected to a single duct and/or nozzle outlet; this may, for example, be spirally grooved on its inner surface in order to enhance the efficiency of mixing.

Alternatively the formulations may be dispensed separately and mixed by the patient. Thus, for example, a dispense system of the type described in the above-mentioned EP-A-0644129 may be fitted with separate duct/nozzle outlets for dispense of each formulation. Separate operation of each actuator will deliver appropriate relative amounts of the formulations, e.g. onto the hand or directly onto an affected area of skin; the formulations may then be mixed by the patient, e.g. by rubbing. It will be appreciated that such embodiments may be preferred to products which include mixing means in cases where the active ingredients are mutually reactive to the extent that they may generate toxic or otherwise undesirable by-products while residual mixed formulations stand in a common duct and/or nozzle between successive dispense operations.

The dispense means for use in the invention may comprise a dual- or multi-chamber dispense system as hereinbefore described which is provided with a removable closure (e.g. a cap). Preferably, in use, the closure prevents the formulations from being accidentally dispensed. For example, in the case of a system which comprises an actuator (e.g. a plunger) which must be depressed to deliver the product, the closure may fit over the actuator thereby physically preventing this from being depressed when the dispense system is not in use, e.g. when being carried in a handbag.

The closure may also function to prevent exposure of the orifice(s) of the dispenser to the air when the product is not in use. For example, this may act as a temporary cover or seal for the orifice(s) from which the individual components of the product are dispensed thus preventing (or, at least, reducing) exposure of these to the air. This function of the closure is particularly important in the case of water-based or aqueous formulations as herein described which on exposure to the air have a tendency to evaporate and dry out. Since this can result in the formation of a caked layer or crust on the surface of any cream or lotion which remains in the dispenser, exposure to the air should be minimised when the product is not in use. Any drying out of the cream or lotion within the duct(s) and/or external orifice(s) of the dispenser may also reduce the size of the duct(s) and/or orifices (s).

This, in turn, may affect the amount of product dispensed in any given operation. In some cases, exposure of the product to the air may even result in complete blockage of the duct(s) and/or dispense orifice (s) .

The closure for use in the invention may, for example, be provided with one or more (preferably, a plurality) of protrusions. These will generally correspond in number to the number of orifices provided in the dispenser. Typically, the closure may be provided with 1, 2 or 3 protrusions, preferably 2. When the closure is in place on the dispenser these protrusions engage with the orifices of the dispenser. In order to provide a temporary seal, these protrusions will generally provide a close fit with each orifice and, typically, will be shaped complimentarily to them. Thus, for example, where the orifices are circular in shape, the protrusions will have a circular cross-section, e.g. they will be generally cylindrical. One or more downward flanges (e.g. teeth) may also be provided on the sides of the closure for the purposes of alignment and final placement onto the dispenser.

The closure may be completely or partly (e.g. by pivoting) removed from the dispenser prior to dispense of the product and readily fits (e.g. snaps) back into place once the product has been dispensed.

The closure may be made of any suitable material but will generally comprise a plastics material, e.g. polypropylene or polyethylene.

Viewed from a further aspect the invention thus provides a closure (e.g. a cap) for a dual- or multi-chamber dispense system having one or more, preferably a plurality, e.g. 2, orifices from which separately stored formulations can be dispensed, wherein said closure comprises one or more, preferably a plurality, e.g. 2, protrusions which are complimentary in shape to said orifices. Preferably, the closure is further adapted such that in use this prevents the formulations from being dispensed. For example, this may be adapted to prevent depression of an actuator or plunger. More preferably, the closure is adapted to cover, e.g. to seal, the orifice or orifices of the dispenser when this is not in use.

In another aspect the invention provides a dual or multi-chamber dispense system for dispensing separately stored formulations which comprises an actuator which causes dispense of said formulations from one or more (preferably a plurality, e.g. 2) orifices, wherein said system is further provided with a closure which prevents depression of said actuator. Preferably, when in use, the closure also acts as a cover or seal to the orifice(s) of the dispenser.

Preferably, the dispense system and/or cap in accordance with the invention have one or more of the advantageous features discussed above.

Preferred embodiments of these aspects of the invention are described with reference to the accompanying Figures, in which:
Figure 1A shows a conventional multi-chamber dispenser;
Figure 1B shows a conventional multi-chamber dispenser provided with a cap in accordance with the invention;
Figure 2 shows a cap in accordance with the invention; and
Figure 3 shows a conventional multi-chamber dispenser which is provided with a cap in accordance with the invention which is partially removed.

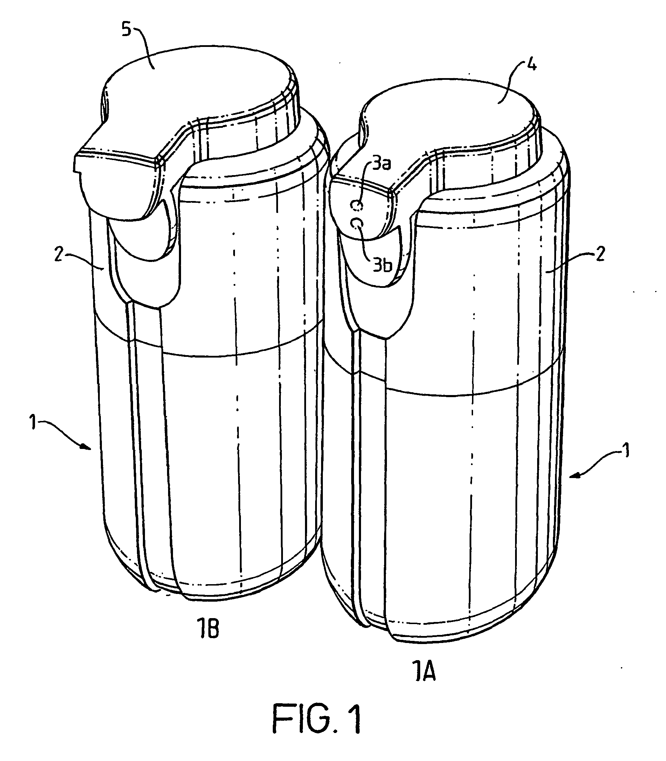

With reference to Figure 1A, a container 1 for a multi-component product has a body 2 within which are located first and second chambers (not shown) suitable for storing first and second formulations. Each chamber is provided with a duct (not shown) connecting it to an orifice 3a and 3b. There is further provided a dispensing mechanism which comprises an actuator 4 which is connected to a pumping mechanism of conventional design and which is not discussed further herein. Depression of the actuator 4 delivers appropriate amounts of the first and second formulations to the affected area of the skin via the orifices 3a and 3b.

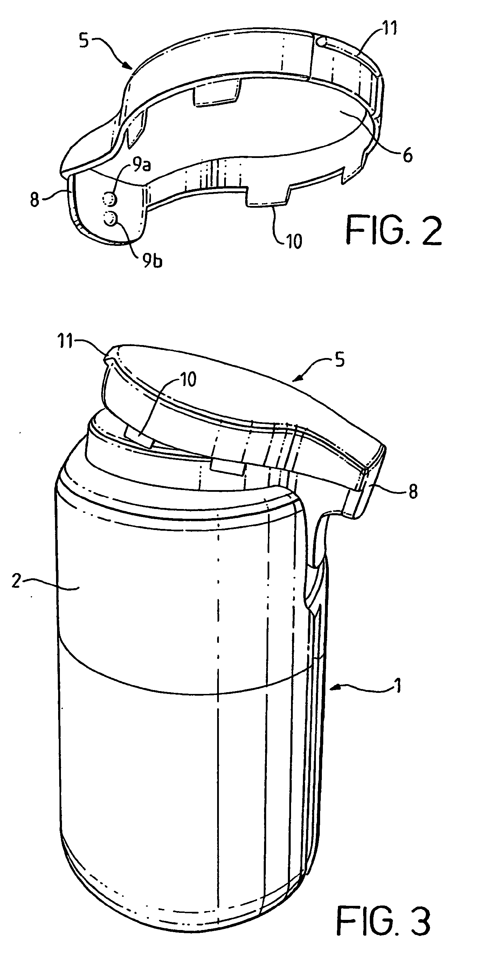

The product of the invention is similar to that known in the art but is additionally provided with a cap 5. As shown in Figure 2, cap 5 comprises a generally circular skirt 6 having a depending rim 7. At the front of the skirt 6 and depending from the front portion is a novel cover 8 which on an inner face is provided with two pips 9a and 9b. These pips 9a and 9b are complimentary in shape to the orifices 3a and 3b of the container. The cap is additionally provided with four engagement members or teeth 10 which depend from the rim 7. A notch 11 is also provided at the rear portion of the skirt 6.

In use, cap 5 engages with the top of the container 1 and generally fits over the actuator 4. The teeth 10 form an interference fit between the actuator 4 and the body 2 of the container 1 thereby securing the cap 5 in place. The two pips 9a and 9b engage in the orifices 3a and 3b and function not only to prevent depression of the actuator 4 but also to seal the orifices 3a and 3b from the air. In operation, notch 11 at the rear of the cap 5 is pushed upwards with the thumb for easy removal of the cap 5. Actuator 4 is depressed and the product is dispensed. The cap 5 is then simply replaced on the container 1.

In one class of preferred products according to the invention, the first formulation is a water-based (e.g. aqueous) topical cream or gel carrier base containing an antibacterial and/or keratolytic agent incorporated into a polymeric delivery system, and the second is a water-based (e.g. aqueous) carrier base having substantially the same lipophilicity and containing a topical antibiotic; if desired, the antibiotic may also be contained within a polymeric delivery system.

Preferred antibacterial agents include salicylic acid and organic peroxides, especially benzoyl peroxide. Combinations of antibacterials, such as salicylic acid and organic peroxides, e.g. salicylic acid and benzoyl peroxide, may also be used. Salicylic acid is particularly suitable for use in treating acne, e.g. acne vulgaris, since it is both an antibacterial and a keratolytic agent and may, for example, be present in such a first formulation in an amount of 0.2-40% w/w, advantageously 1-30% w/w, preferably 2-10% w/w. For treating acne vulgaris, appropriate concentrations of salicylic acid may, for example, be 0.5% w/w or 2% w/w. Organic peroxides may, for example, be present in amounts of 0.2-40% w/w, advantageously 2-30% w/w, preferably 2.5-20% w/w. Combinations of salicylic acid and benzoyl peroxide may advantageously comprise 2% w/w salicylic acid in combination with 2.5% or 5% w/w benzoyl peroxide.

Other keratolytic agents which may be used include retinoids such as retinol or retinoic acid and salts and esters thereof, for example in amounts of 0.01-2% w/w, advantageously 0.025-1% w/w, preferably 0.02-0.2% w/w. Preferred retinoids include retinoic acid, isotretinoin, tretinoin, retinol, retinyl palmitate, adapalene, tazarotene and azelaic acid. When applied topically, azelaic acid helps to normalise keratinization, to reduce the proliferation of *P. acnes* and is effective against both non-inflammatory and inflammatory acne lesions. Its efficacy can be enhanced when used in combination with benzoyl peroxide, clindamycin, tretinoin or erythromycin/benzoyl peroxide.

A preferred combination of antibacterial and keratolytic agents which may be present in the first formulation is benzoyl peroxide together with retinoic acid or retinol.

It will be appreciated that the amount of antibacterial and/or keratolytic agent (and of all other active ingredients) should be selected to give a desired end product concentration following the overall dilution of each ingredient which will occur when the formulations are mixed.

Polymeric delivery systems useful in products of the invention will generally comprise a polymer or polymers in the form of particles (e.g. microparticles), aggregates of particles (e.g. aggregates of microparticles) or clusters of aggregates (agglomerates) of particles (e.g. agglomerates of microparticles) which are capable of entrapping any desired active for delayed release. The polymer particles will generally be porous (i.e. these have an open structure) and will also typically be cross-linked, e.g. comprising a porous polymeric matrix. Examples of polymeric delivery systems suitable for use in the invention include the Poly-Trap^{®} (Cardinal Health, Inc.) and Poly-Pore^{®} (Amcol International, Inc.) systems and, in particular, the Microsponge^{®} system (Advanced Polymer Systems, Inc.).

Poly-Pore is a microparticle delivery system comprising allyl methacrylate cross-polymer and is described, for example, in US-A-5830960, US-A-5834577 and US-A-6248849. It comprises spherical particles having a median particle size of about 30 µm. The interior of the Poly-Pore spheres comprises clusters of small spheres which are then agglomerated together to form a porous exterior surface and a hollow interior. Poly-Pore has the unique ability to adsorb both hydrophobic and hydrophilic liquids.

The Poly-Trap family of compounds are made by a process which involves supsension polymerization of lauryl methacrylate and ethylene glycol dimethacrylate using a peroxide as a catalyst. The polymerization conditions lead to the formation of amorphous microaggregates or polymer particles. The size of these aggregates is about 25 µm in diameter and within their structure is a high degree of cross-linking. The Poly-Trap polymers are extremely lipophilic and hydrophobic. As a result, they are ideal carriers for lipophilic actives and are also capable of controlling skin oiliness without dehydrating the skin. Poly-Trap polymers suitable for use in the products herein described are, for example, described in US-A-4962133 and US-A-4962170.

Microsponge delivery systems can be made, for example, using either styrene and divinylbenzene, or methyl methacrylate and ethylene glycol dimethyacrylate as starting materials. The choice of monomers and cross-linkers enable different families of compounds to be prepared. Whilst these both consist of porous microspheres their physical-chemical properties are quite different. Furthermore, the manufacturing process used to produce Microsponge products allows these to be produced with a wide range of particle size, porosity (void volume), pore diameter (openings on the surface) and surface area. With an almost unlimited number of variations, this permits customization of the polymer to the active ingredient to be entrapped. Specifically, this allows design and control of the required release rate and maximisation of beneficial effects on the skin.

Microsponge systems useful in products of the invention may, for example, be as described in WO-A-88/10132, US-A-4873091, US-A-4690825 and EP-A-0306236. Thus, for example, antibacterial agents such as benzoyl peroxide may be formulated in similar manner to the product marketed in the USA under the tradename Exact^{®} and by the present applicant in Turkey under the name Aksil^{®}; benzoyl peroxide-containing Microsponges are described in, for example, US-A-5879716. Similar Microsponges containing retinoic acid are described in US-A-5955109. Loading of the active ingredient may take place via either a one-step or two-step process, e.g. as described in US-A-4690825 and US-A-5145675 respectively, whereafter the resulting Microsponges may be suspended in the desired carrier base (e.g. an aqueous carrier base).

An advantage of the polymeric delivery systems herein described is that release of the antibacterial and/or keratolytic agent from the system (e.g. from the Microsponges) can be made to occur quite slowly, conveniently with onset being triggered by dispense and/or application with rubbing of the formulations onto the skin. The concentration of the agent in the carrier base at any time may consequently be low, minimising possible irritant side-effects whilst maintaining a therapeutically effective concentration. Similar advantages may accrue if the topical antibiotic is also contained within its own polymeric delivery system.

The particles of the polymeric delivery system may be composed of a wide range of materials, including both synthetic polymers and natural substances such as cellulose or gelatin. The choice of material forming the polymeric delivery system may depend upon the intended methods by which the entrapped antibacterial or other agent is to be released. Such methods are described in J. Microencapsulation (1996), 13 (5), 575-588 and include but are not limited to diffusion, compression, dissolution or melting.

Preferably, entrapped antibacterial and/or keratolytic agent is released from the polymeric delivery system by diffusion from the pores of the polymeric particles into the carrier. The rate of diffusion will depend on the partition coefficient of the antibacterial and/or keratolytic agent (and any other entrapped active ingredients) between the polymer forming the polymeric delivery system and the carrier.

Porous particles containing agents such as benzoyl peroxide or retinoic acid will typically release sufficient of the agent into the carrier base during storage, dispense and/or application to provide a therapeutically effective initial concentration of agent in the formulation as applied to the skin. Agents such as salicylic acid, however, may not undergo such ready initial release from a polymeric delivery system because of their different lipophilicity; it may therefore be advantageous to include a proportion of "free" active agent in the carrier base (e.g. up to 25% w/w of the total content of the agent) to provide the required initial therapeutically effective concentration and to further induce release of agent from the polymeric particles (e.g. microsponges).

The diameter of the porous particles which comprise the polymeric delivery system may, for example, be in the range 1 to 1000 microns, e.g. 4 to 300 microns, such as 5 to 100 microns. It is preferred, however, that the particle size is less than 30 microns, e.g. 10 to 25 microns, since particles larger than 30 microns can impart a 'gritty' feel to the formulation, which may decrease patient compliance.

The surface area of the porous particles which comprise the microsponge delivery system may, for example, range from 1 to 500 m²/g, e.g. 20 to 200 m²/g; the total pore volume may, for example, be in the range 0.3 to 4.0 cm³/g, e.g. 0.6 to 2.0 cm³/g and the pore diameter (i.e. the opening of the pores on the surface of the particle) may range from 0.001 to 1 micron, e.g. from 0.01 to 0.1 micron. Pore volume and, more importantly, pore diameter may have a significant effect on the rate of release of the entrapped antibacterial and/or keratolytic agent, and may affect the migration of the agent from the polymeric delivery system into the carrier in which the polymeric delivery system is dispersed. Thus the diameter (and hence volume) of the pores has a direct impact on the release of the agent, as well as on the amount of agent that can be entrapped within the delivery system.

The polymeric delivery system may be made by suspension polymerisation, preferably crosslinking polymers such as polyolefins, for example polyethylene, polystyrene and polydicyclopentadiene; polyacrylate esters, for example optionally alkoxylated C₁₋₁₀ alkyl, cycloalkyl, aryl or aralkyl esters of polyacrylic or polymethacrylic acids; polyvinyl esters, for example polyvinyl acetate or polyvinyl laurate; polyvinyl ketones, for example polyvinylmethyl ketone; and polyvinyl ethers, for example polyvinyl propyl ether. The most commonly used crosslinking agents are divinylbenzene for polystyrene polymers and ethylene glycol dimethacrylate for polymethacrylates.

It will be appreciated that the level of hardness of the particles of the polymeric delivery system may be varied widely by appropriate selection of the polymer composition, degree of crosslinking etc. It is desirable that the particles are elastically compressible so that after application of the formulation to an affected area, the application of gentle pressure, for example by rubbing, may induce release of the entrapped antibacterial and/or keratolytic agent into the carrier and thus to the skin.

In addition to the antibacterial and/or keratolytic agent, the porous microspheres of the polymeric delivery system may entrap a wide range of other ingredients such as emollients, fragrances, antioxidants, essential oils, sun screens, anti-infective, antifungal and anti-inflammatory agents, more particularly fragrances and antioxidants such as butylated hydroxyl anisole, butylated hydroxyl toluene, alkyl gallates (e.g. propyl gallate), or tocopherols.

Preferred topical antibiotics useful in the second formulation of the aforementioned class of preferred products include tetracyclines (e.g. formulated at concentrations of 0.2-20% w/w, advantageously 1-12% w/w, preferably 2-4% w/w), erythromycin (e.g. formulated at concentrations of 2-30% w/w, advantageously 3-20% w/w, preferably 5-10% w/w), and clindamycin (e.g. formulated at concentrations of 0.02-20% w/w, advantageously 0.2-10% w/w, preferably 1.6-5.2% w/w). Again allowance should be made for the overall dilution of individual active ingredients which will occur when the formulations are mixed. Where appropriate, e.g. for solubility or distribution considerations, corresponding salts or esters, e.g. mineral acid addition salts such as clindamycin hydrochloride or phosphate, or carboxylic acid esters such as erythromycin propionate, stearate or ethylsuccinate may also be used. Clindamycin phosphate is particularly suitable for use in formulations used for acne control (*P. acnes* is highly sensitive to clindamycin; clindamycin is active in comedones from acne patients; it also reduces free fatty acids on the skin surface).

Tetracyclines suitable for use in the second formulation include, in particular, tetracycline, doxycycline, minocycline and lymecycline.

The term "water-based carrier" as used herein denotes any topical carrier in which water is present, preferably in an amount in excess of 5% w/w, e.g. in excess of 10, 20, 30 or 40% w/w. The term "aqueous carrier base" is intended to denote any topical carrier base in which water is the major component, e.g. being present in amounts in excess of 50, 55, 60, 65 or 70% w/w. Carrier bases contemplated for use in the invention include aqueous creams, gels, lotions or ointments, as well as oil-in-water and water-in-oil emulsions. Water-in-oil emulsions, for example, enable the delivery of hydrophilic ingredients from the polymeric delivery system.

The carriers may, for example, include conventional formulating ingredients selected from lipophilic base materials (for example fatty (e.g. C₁₀₋₃₀) alcohol esters of saturated or unsaturated fatty (e.g. C₁₀₋₃₀) acids, such as cetyl ricinoleate; fatty acid esters of sterols such as cholesterol or lanosterol; emollient silicon oils, e.g. polysiloxanes such as dimethicone or cyclomethicone; or terpenes such as α-bisabolol), hydrophilic base materials (for example polyethylene glycols, hereinafter referred to as PEGs), stabilisers and/or surfactants (for example fatty acids such as palmitic or stearic acid; fatty alcohols such as cetyl or stearyl alcohol; amphiphilic fatty esters, e.g. fatty alcohol esters of mineral acids such as sodium lauryl sulphate, fatty acid esters of polyols such as glyceryl dilaurate or caprylic/capric triglyceride; PEGylated fatty alcohols, e.g. PEG lauryl ethers such as laureth-4; PEGylated sorbitan esters with fatty acids such as oleic, lauric, palmitic or stearic acid, e.g. as in Tween^{®} surfactants; PEGylated sterols such as PEG-10 soya sterol; polysaccharides such as xanthan gum; proprietary products such as emulsifying wax; or thickening polymeric stabilisers, e.g. polyacrylamide-based products such as Sepigels^{®}), humectants (for example diols or polyols such as propylene glycol or glycerol), viscosity modifiers (for example saccharides such as sorbitol), thickeners (for example colloidal or fumed silica or silicates such as magnesium aluminium silicate), preservatives (for example antimicrobials or antifungals such as methyl paraben, propyl paraben, benzyl alcohol, phenoxyethanol or germaben II; or antioxidants such as vitamin E, ascorbyl palmitate or butylated hydroxytoluene), pH regulators (for example buffers, e.g. acid/salt combinations such as citric acid/sodium citrate; or bases such as triethanolamine), or anticoagulants (for example disodium edetate).

As noted above, the lipophilicity of a formulation affects the partition coefficient of active ingredient contained within a polymeric delivery system between the polymer particles and the carrier. The requirement for the carriers within a particular product according to the invention to have substantially the same lipophilicity may therefore be tested and quantified by determining the partition coefficient in respect of each carrier base; the requirement is met if the partition coefficients vary by no more than 10%, advantageously by no more than 5%, preferably by no more than 2.5%. It is also preferred that the individual water contents and viscosities of the formulations within a particular product vary by no more than these limits, since this may enhance ease and uniformity of mixing of the formulations during or after dispense. In a preferred embodiment of the invention the individual formulations will have substantially identical viscosities, e.g. varying by no more than 10%, preferably by no more than 5%, more preferably by no more than 2.5%.

In general the viscosities of the formulations may be up to 200,000 mPa.s, preferably up to 100,000 mPa.s, more preferably in the range 5,000 to 50,000 mPa.s, yet more preferably 5,000 to 15,000 mPa.s, e.g. about 10,000 mPa.s.

The use of formulations having lower viscosities, e.g. of the order of about 10,000 mPa.s, is particularly preferred when dispensing these from systems having side-by-side chambers or compartments from which the product is dispensed by the application of external pressure means. In particular, it has been found when using such systems that it is important to ensure that each formulation (e.g. cream, gel or lotion) should be free flowing to the extent that this essentially remains at the base of the container where the suction tube orifice is located. This orifice must be kept inside each formulation at all times otherwise air will enter the system causing inaccurate amounts of cream to be dispensed. On the other hand, it is important that the viscosity of each formulation should not be too low since the liquid will not stick to the skin. The use of low viscosity formulations has the added advantage that non-pressurized systems may be used to dispense these. Such systems are more environmentally friendly than those which utilise an inert gas to force gels into the intake tubes of the pump. These are also more cost effective to manufacture.

The carriers within a particular product may advantageously contain essentially the same ingredients or close analogues, homologues or equivalents thereof, in amounts appropriate to ensure the desired levels of lipophilicity etc. Where it is desired substantially to match the viscosities of the formulations within a product, it will be appreciated that the presence of polymer particles (e.g. microsponges) in a formulation may have a significant viscosity increasing effect, particularly if the content of the polymer particle is relatively high (for example as may be required if the level of entrapment of active ingredient in the polymer particles is relatively low). It may therefore be desirable to increase the relative amounts of viscosity enhancing agents (e.g. polysaccharides such as xanthan gum or thickening agents such as silica or silicates) and/or to reduce the amount of relatively low viscosity components such as glycerol or sorbitol in a corresponding non-polymeric particle (e.g non-Microsponge) formulation in order to compensate for this. Alternatively, both formulations may include polymer particles (e.g. Microsponges) in order to match the viscosity levels.

Whilst the invention is primarily described herein in terms of systems comprising oxidising antibacterials (e.g. benzoyl peroxide) and antibiotics, as noted above the invention is more generally applicable to any topical pharmaceutical and/or cosmetic preparation which involves two or more formulations, at least one of which involves a polymeric delivery system. Such preparations need not necessarily involve components which have stability limitations when combined. For example, the product herein described may be suitable for use with any conventional dermatological preparation, e.g. any topical anti-acne compositions. Such compositions may include, for example, one or more of the following active components: organic peroxides (e.g. benzoyl peroxide), retinoids (such as retinoic acid, retinol, tretinoin, isotretinoin, adapalene and tarazotene, e.g. at a concentration of 0.001-1% w/w, preferably 0.025-1% w/w, more preferably 0.05-1% w/w), other comedolytic agents (e.g. azelaic acid and salicylic acid), sulfur, resorcinol, zinc, anti-inflammatory steroids (such as corticosteroids, e.g. hydrocortisone, which may be present in concentrations in the range 0.25-2.5% w/w), antibiotics (e.g. clindamycin, erythromycin, tetracycline, doxycycline, minocycline, lymecycline), anti-fungal agents (e.g. undecilenic acid, miconazole (base and nitrate), ketoconazole, iconazole, clotrimazole and metronidazole), alpha-hydroxy acids (e.g. glycolic acid, lactic acid, kojic acid), vitamin K, hair growth promotion agents (such as minoxidil, e.g. present in a concentration of 1-5% w/w), depigmenting agents (such as hydroquinone, e.g. at a concentration of 1-10% w/w), antiviral agents (e.g. acyclovir, valacyclovir and the like or a combination thereof), anaesthetic agents (e.g. lidocaine, benzocaine, prilocaine, pramoxine, benzyl alcohol, dibucaine), counter-irritant agents (e.g. menthol, camphor, phenol and the like or a combination of these), antiseptic/microbicidal agents (e.g. benzalkonium chloride, boric acid, cetylpyridinium chloride, benzethonium chloride, resorcinol, chloroxyenol and the like), antibacterial and/or antitrichomonal agents (e.g. metronidazole, tinidazole, ornidazole and the like), astringents (e.g. bismuth subgallate, zinc oxide, witch hazel and the like), wound healing agents (e.g. cod liver oil, shark liver oil, sucralfate and the like), protectants (e.g. dimethicone, aluminium hydroxide gel, aluminium acetate, mineral oil, cocoa butter, glycerine, kaolin, petrolatum, lanolin oil, peruvian balsam, calamine, titanium dioxide, etc.), hormones (e.g. conjugated estrogens, estriol and the like), vitamins (e.g. vit. A, vit. D, vit. E, vit. C and the like), hyaluronidase, topical analgesic agents (e.g. capsaicin, methyl salicylate and the like), topical non-steroidal anti-inflammatory agents (nimesulide, diclofenac, ibuprofen, piroxicam, ketoprofen, etc.), topical antihistaminic agents (e.g. diphenhydramine, tripelennamine and the like), insect repellants (e.g. n,n-diethyl-meta-toluamide and other isomers, octyl methoxycinnamate, oxybenzone), pediculicides (e.g. piperonyl butoxide, pyrethrins, phenotrineburn etc.), sunburn agents, sunscreen and suntan products (e.g. octylmethoxycinnamate, octocrylene, octyl salicylate, benzophenone, melanine and the like).

As will be appreciated by those skilled in the art, the amount of any active or actives present in the formulations for use in the invention will be selected such that this provides a desired concentration in the final mixed product (i.e. this should be determined taking into account the dilution of the active which will occur on mixing of the separate formulations).

Non-limiting examples of dermatological and/or cosmetic formulations suitable for use in the invention include the following:

| **First formulation** | **Second formulation** |
|---|---|
| Salicylic Acid* | Clindamycin |
| Clindamycin | Salicylic Acid* |
| Benzoyl peroxide* | Erythromycin |
| Benzoyl peroxide* | Clindamycin phosphate |
| Benzoyl peroxide* | Tazarotene |
| Benzoyl peroxide* | Salicylic acid |
| Tretinoin* | Clindamycin |
| Azelaic Acid* | Clindamycin |
| Azelaic Acid* | Erythromycin |
| Benzoyl Peroxide* | Azelaic Acid |
| Erythromycin | Azelaic Acid* |
| Azelaic Acid* | Minocycline |
| Azelaic Acid | Tretinoin* |
| Tazarotene* | Clindamycin |
| Benzoyl Peroxide* | Tazarotene* |
| Benzoyl Peroxide/ | |
| Erythromycin | Tazarotene* |
| Zinc Sulphate | Erythromycin* |
| Zinc Sulphate | Clindamycin* |
| Antibiotic | Hydrocortisone* |
| Benzoyl Peroxide* | Hydrocortisone/ antibiotic |
| Hydrocortisone* | Metronidazole |
| Resorcinol* | Sulfur |
| Salicylic Acid* | Hydroquinone* |
| Retinoid* (e.g. retinol) | Corticosteroid* (e.g. hydrocortisone) |
| Retinoid* | Hydroquinone* |
| Retinoid/Corticosteroid* | Hydroquinone* |
| Retinoid* | Antifungal* |
| Retinoic Acid | Antibiotic* |
| Retinoic Acid | Benzoyl Peroxide* |
| Retinoic Acid | Salicylic Acid* |
| Retinoic Acid* | Antibiotic* |
| Retinoic Acid* | Benzoyl Peroxide* |
| Retinoic Acid* | Salicylic Acid* |

| | |
|---|---|
| * signifies presence in a polymeric delivery system, preferably in a Microsponge system. | |

Particularly preferred combinations of actives for use in products of the invention include: salicylic acid and clindamycin (e.g. clindamycin phosphate); benzoyl peroxide and clindamycin; benzoyl peroxide and salicylic acid; and retinoic acid (or a derivative thereof) and an antibiotic (e.g. clindamycin). In these particular combinations, either of the actives may 'entrapped', i.e. present in a polymeric delivery system, preferably in a Microsponge system. However, preferably it will be the first listed active which will be 'entrapped'.

Combinations of retinoic acid (or a derivative thereof) either in 'entrapped' or 'free' form together with an 'entrapped' form of an antibiotic, benzoyl peroxide or salicylic acid are also preferred for use in the invention.

The pharmaceutical formulations in products of the present invention may be manufactured by methods conventionally known for the manufacture of pharmaceutical creams or gels. One suitable method of manufacture includes the steps of preparing an aqueous solution of the water-soluble ingredients, mixing this solution together with the hydrophobic ingredients, homogenising the resulting mixture and thereafter adding the active ingredient (e.g. antibacterial agent or antibiotic). The resulting cream or gel may then be filled into the appropriate storage means of the product.

To use a product of the present invention, a patient may simply activate the dispense means (e.g. by depressing a pump or plunger) and collect the formulation(s) dispensed, e.g. in his hand. If necessary, the patient may then mix the formulations to obtain a combination product which is applied to the area(s) of skin to be treated. Formulations dispensed from the products of the invention are preferably applied on a regular basis, for example once or twice daily.

Although much of the discussion and examples presented herein pertain to products primarily designed for the treatment of acne conditions, it will be clear to those of ordinary skill in the art that similar considerations and formulation strategies are equally applicable to the treatment of a wide range of other skin conditions. For example, the products of the invention may be used in treating other dermatological conditions, such as fungal infections, various dermatitis (e.g. eczema, psoriasis and the like), rosacea, melasma (chloasma) and alopecia. Such products also find use in non-medical, e.g. cosmetic, applications such as in treating or preventing wrinkles, in treating rough skin, hyperpigmented spots (e.g. liver spots) and other manifestations of aging skin, cellulite, stretch marks, etc., and in promoting or suppressing hair growth.

The products herein described may also be used in treating dermatological conditions and conditions affecting mucosal surfaces (e.g. skin & mucosal & oral membranes) and its appendices, such as pseudofolliculitis barbae, fungal infections, bacterial infections, viral infections, allergic and/or atopic dermatitis (eczema) such as seborrheic dermatitis, diaper rash and the like, conditions related to wounds, burns, sports injuries, insect bites, sunburn and the like, as well as infectious and non-infectious conditions affecting skin & mucosal membranes of the genital area such as vulvitis, balanitis and the like, and hemorrhoidal conditions.

The invention will now be described in more detail by way of the following non-limiting Examples:

### Example 1 - Benzoyl Peroxide/Salicylic Acid Lotion in Double Container (Standard Strength Formulation)

### 5% Benzoyl Peroxide Lotion:

| Component | % by weight |
|---|---|
| 1. Deionized Water | 8.62 |
| 2. Disodium EDTA | 0.05 |
| 3. Aloe Vera Gel | 2.50 |
| 4. Carbowax PEG-1450 | 6.00 |
| 5. Panthenol 50P | 0.30 |
| 6. Allantoin | 0.10 |
| 7. Germaben II | 1.00 |
| 8. Amigel 3% Aq. Soln. | 50.0 |
| 9. Propylene Glycol | 5.00 |
| 10. Briz-30 | 5.50 |
| 11. Tween 80 | 2.50 |
| 12. Dimethicone | 6.00 |
| 13. Benzoyl Peroxide Entrapment | 11.63 |
| 14. Sepigel 305 | 1.00 |

### Preparation:

1. Disperse component 2 in deionised water and add components 3, 4, 5, 6 and 7 with mixing.
2. Prepare a premix of component 8 and add to above with mixing.
3. Add components 9-13 with slow mixing.
4. Add component 14 with mixing.

### 3% Salicylic Acid Lotion:

| Component | % by weight |
|---|---|
| 1. Deionized Water | 9.71 |
| 2. Disodium EDTA | 0.05 |
| 3. Aloe Vera Gel | 2.50 |
| 4. Carbowax PEG-1450 | 6.00 |
| 5. Panthenol 50P | 0.30 |
| 6. Allantoin | 0.10 |
| 7. Germaben II | 1.00 |
| 8. Amigel 3% Aq. Soln. | 50.0 |
| 9. Propylene Glycol | 5.00 |
| 10. Briz-30 | 5.50 |
| 11. Salicylic Acid | 0.50 |
| 12. Tween 80 | 2.50 |
| 13. Dimethicone | 6.00 |
| 14. Salicyclic Acid Entrapment | 9.54 |
| 15. Sepigel 305 | 1.30 |

### Preparation:

1. Disperse component 2 in deionised water and add components 3, 4, 5, 6 and 7 with mixing.
2. Prepare a premix of component 8 and add to above with mixing.
3. Add components 9-14 with slow mixing.
4. Add component 15 with mixing.

The Benzoyl Peroxide and Salicylic Acid Formulations are subsequently transferred to a dual-chamber dispense system such as that described in EP-A-0644129 which may be provided with a suitable cap as herein described.

### Example 2 - Benzoyl Peroxide/Salicylic Acid Lotion in Double Container (Extra Strength Formulation)

### 7% Benzoyl Peroxide Lotion:

| Component | % by weight |
|---|---|
| 1. Deionized Water | 4.05 |
| 2. Disodium EDTA | 0.05 |
| 3. Aloe Vera Gel | 2.50 |
| 4. Carbowax PEG-1450 | 6.00 |
| 5. Panthenol 50P | 0.30 |
| 6. Allantoin | 0.10 |
| 7. Germaben II | 1.00 |
| 8. Amigel 3% Aq. Soln. | 50.0 |
| 9. Propylene Glycol | 5.00 |
| 10. Briz-30 | 5.50 |
| 11. Tween 80 | 2.50 |
| 12. Dimethicone | 6.00 |
| 13. Benzoyl Peroxide Entrapment | 16.30 |
| 14. Sepigel 305 | 1.00 |

### Preparation:

1. Disperse component 2 in deionised water and add components 3, 4, 5, 6 and 7 with mixing.
2. Prepare a premix of component 8 and add to above with mixing.
3. Add components 9-13 with slow mixing.
4. Add component 14 with mixing.

### 4% Salicylic Acid Lotion:

| Component | % by weight |
|---|---|
| 1. Deionized Water | 5.6751 |
| 2. Disodium EDTA | 0.05 |
| 3. Aloe Vera Gel | 2.50 |
| 4. Carbowax PEG-1450 | 6.00 |
| 5. Panthenol 50P | 0.30 |
| 6. Allantoin | 0.10 |
| 7. Germaben II | 1.00 |
| 8. Amigel 3% Aq. Soln. | 50.0 |
| 9. Propylene Glycol | 5.00 |
| 10. Briz-30 | 5.50 |
| 11. Salicylic Acid | 0.50 |
| 12. Tween 80 | 2.50 |
| 13. Dimethicone | 6.00 |
| 14. Salicyclic Acid Entrapment | 13.40 |
| 15. Sepigel 305 | 1.50 |

### Preparation:

1. Disperse component 2 in deionised water and add components 3, 4, 5, 6 and 7 with mixing.
2. Prepare a premix of component 8 and add to above with mixing.
3. Add components 9-14 with slow mixing.
4. Add component 15 with mixing.

The Benzoyl Peroxide and Salicylic Acid Formulations are subsequently transferred to a dual-chamber dispense system such as that described in EP-A-0644129 which may be provided with a suitable cap as herein described.

### Example 3 - Clindamycin + Benzoyl Peroxide (or Salicylic Acid or Retinoic Acid) Lotion in Double Container

### 2% Clindamycin Lotion (Entrapped):

| Component | % by weight |
|---|---|
| 1. Deionized Water | 13.89 |
| 2. Disodium EDTA | 0.05 |
| 3. Aloe Vera Gel | 2.50 |
| 4. Carbowax PEG-1450 | 6.00 |
| 5. Panthenol 50P | 0.30 |
| 6. Allantoin | 0.10 |
| 7. Germaben II | 1.00 |
| 8. Amigel 3% Aq. Soln. | 50.0 |
| 9. Propylene Glycol | 5.00 |
| 10. Briz-30 | 5.50 |
| 11. Clindamycin | 0.33 |
| 12. Tween 80 | 2.50 |
| 13. Dimethicone | 6.00 |
| 14. Clindamycin Entrapment | 5.53 |
| 15. Sepigel 305 | 1.30 |

### Preparation:

1. Disperse component 2 in deionised water and add components 3, 4, 5, 6 and 7 with mixing.
2. Prepare a premix of component 8 and add to above with mixing.
3. Add components 9-14 with slow mixing.
4. Add component 15 with mixing.

The Clindamycin formulation may be used in combination with any of the Benzoyl Peroxide and Salicylic Acid formulations according to Examples 1 and 2 or with a 0.05 wt% Retinoic Acid formulation. The components are presented in a dual-chamber dispense system such as that described in EP-A-0644129 and which may be provided with a suitable cap as herein described.

### Example 4 - Clindamycin + Benzoyl Peroxide (or Salicylic Acid or Retinoic Acid) Lotion in Double Container

### 2% Clindamycin Lotion (Free Clindamycin):

| Component | % by weight |
|---|---|
| 1. Deionized Water | 17.75 |
| 2. Disodium EDTA | 0.05 |
| 3. Aloe Vera Gel | 2.50 |
| 4. Carbowax PEG-1450 | 6.00 |
| 5. Panthenol 50P | 0.30 |
| 6. Allantoin | 0.10 |
| 7. Germaben II | 1.00 |
| 8. Amigel 3% Aq. Soln. | 50.0 |
| 9. Propylene Glycol | 5.00 |
| 10. Briz-30 | 5.50 |
| 11. Clindamycin | 2.00 |
| 12. Tween 80 | 2.50 |
| 13. Dimethicone | 6.00 |
| 14. Sepigel 305 | 1.30 |

### Preparation:

1. Disperse component 2 in deionised water and add components 3, 4, 5, 6 and 7 with mixing.
2. Prepare a premix of component 8 and add to above with mixing.
3. Add components 9-13 with slow mixing.
4. Add component 14 with mixing.

The Clindamycin formulation may be used in combination with any of the Benzoyl Peroxide and Salicylic Acid formulations according to Examples 1 and 2 or with a 0.05 wt% Retinoic Acid formulation. The components are presented in a dual-chamber dispense system such as that described in EP-A-0644129 and which may be provided with a suitable cap as herein described.

A suitable retinoic acid lotion formulation may be prepared by substituting Retinoic Acid in place of the Salicylic Acid in the 3% Salicyclic Acid Lotion of Example 1 (hereinafter referred to as "reference formulation"). The active ingredient would then be Retinoic Acid with a total concentration of 0.2% which would be diluted after mixing with the second component upon dispensing and mixing to obtain the desired 0.1% final concentration. 1/5 of this amount (i.e. 0.2/5 = 0.04%) would be substituted for the 'free' form of Salicylic Acid in the reference formulation and the remaining 4/5 of 0.2% (i.e. 0.16%) would be 'entrapped'. To obtain 30% entrapment, 0.16/0.3 = 0.53% Retinoic Acid would need to be substituted for the 'entrapped' Salicylic Acid ingredient in the reference formulation. Suitable adjustment of the water content of the formula would need to be made to take the total amount of ingredients up to 100%. For a non-entrapped or 'free' version of the Retinoic Acid formulation, the total of 0.2% should be substituted directly into the Salicylic Acid lotion formula and the water adjustment made for the difference.

For formulas requiring lower concentrations of Retinoic Acid such as 0.075%, 0.05% and 0.01% total active, the same calculations should be carried out taking into account the dilution, entrapment and ratio of 'free' to 'entrapped' product.

## Claims

1. A pharmaceutical and/or cosmetic product comprising first and second active ingredient-containing formulations for topical administration to a patient, wherein said product includes storage means whereby said formulations are maintained separately prior to dispense, together with dispense means which permit said formulations to be dispensed from said storage means, **characterised in that** (i) an active ingredient in at least one of said formulations is contained within a polymeric delivery system comprising porous polymer particles capable of delayed release of said active ingredient and (ii) both of said formulations comprise water-based carrier bases in which the partition coefficient of said active ingredient between said polymer particles and each of the carrier bases varies by no more than 10%, wherein said first and second formulations have viscosities which vary by no more than 10%.

2. A product as claimed in claim 1 wherein said formulations each comprise aqueous carrier bases.

3. A product as claimed in claim 1 or claim 2 wherein an active ingredient in at least one of said formulations is contained within a Microsponge^{®} delivery system.

4. A product as claimed in any one of claims 1 to 3 wherein the dispense means are such as to permit dispense of said formulations in controllable relative amounts.

5. A product as claimed in any preceding claim wherein the storage means comprise side-by-side chambers each equipped with a dispense valve, said valves being operable by adjacently disposed actuators.

6. A product as claimed in any one of claims 1 to 4 wherein the storage means comprise a unit dose pouch having separate parts for each formulation.

7. A product as claimed in any of the preceding claims wherein the dispense means are adapted to dispense said formulations separately.

8. A product as claimed in any of the preceding claims wherein the first formulation is an aqueous topical cream or gel carrier base containing an antibacterial and/or keratolytic agent incorporated into a polymeric (e.g. Microsponge) delivery system which comprises porous polymer particles and the second formulation is an aqueous carrier base containing a topical antibiotic.

9. A product as claimed in claim 8 wherein said keratolytic agent is a retinoid.

10. A product as claimed in claim 9 wherein said retinoid is retinoic acid.

11. A product as claimed in claim 8 wherein said antibacterial agent is salicylic acid or an organic peroxide.

12. A product as claimed in claim 11 wherein said organic peroxide is benzoyl peroxide.

13. A product as claimed in any of claims 8 to 12 wherein said antibiotic is erythromycin, clindamycin or a tetracycline.

14. A product as claimed in claim 13 wherein said antibacterial agent is benzoyl peroxide and said antibiotic is clindamycin.

15. A product as claimed in any of claims 8 to 14 wherein said topical antibiotic is contained within a polymeric (e.g. Microsponge) delivery system which comprises porous polymer particles.

16. A product as claimed in any one of claims 1 to 7 which comprises as active ingredients an anti-fungal agent and a retinoid, at least one of which is incorporated into a polymeric (e.g. Microsponge) delivery system which comprises porous polymer particles.

17. A product as claimed in claim 16 wherein the anti-fungal agent is undecilenic acid, miconazole (base or nitrate), ketoconazole, iconazole, clotrimazole and the retinoid is either retinol or retinoic acid.

18. A product as claimed in any one of claims 1 to 7 which comprises as active ingredients a depigmenting agent (e.g. hydroquinone) and a keratolytic agent (e.g. salicylic acid), at least one of which is incorporated into a polymeric (e.g. Microsponge) delivery system which comprises porous polymer particles.

19. A product as claimed in any one of claims 1 to 7 which comprises as active ingredients a depigmenting agent and a retinoid, at least one of which is incorporated into a polymeric (e.g. Microsponge) delivery system which comprises porous polymer particles.

20. A product as claimed in claim 19 which further comprises a corticosteroid, e.g. hydrocortisone.

21. A product as claimed in any one of claims 1 to 7 which comprises as active ingredients a corticosteroid (e.g. hydrocortisone) and a retinoid (e.g. retinol), at least one of which is incorporated into a polymeric (e.g. Microsponge) delivery system which comprises porous polymer particles.

22. A product as claimed in any one of claims 1 to 7 which comprises as active ingredients a hair growth promoter (e.g. minoxidil) and a retinoid (e.g. retinol) or other keratolytic agent, at least one of which is incorporated into a polymeric (e.g. Microsponge) delivery system which comprises porous polymer particles.

23. A product as claimed in any of the preceding claims wherein said first and second formulations have substantially the same water content.

24. A product as claimed in any preceding claim comprising more than two active ingredient-containing formulations (first formulation, second formulation, third formulation, etc.) for topical administration to a patient, together with storage means whereby each of said formulations is separately maintained prior to dispense, wherein (i) an active ingredient in at least one of said formulations is contained within a polymeric delivery system comprising porous polymer particles capable of delayed release of said active ingredient and (ii) all of said formulations comprise water-based carrier bases in which the partition coefficient of said active ingredient between said polymer particles and each of the carrier bases varies by no more than 10%, wherein all of said formulations have viscosities which vary by no more than 10%.

25. A product as claimed in any preceding claim which comprises a dual or multi-chamber dispense system provided with an actuator which causes dispense of said formulations from one or more (preferably a plurality, e.g. 2) orifices, **characterised in that** said system is further provided with a closure which prevents depression of said actuator.

26. A product as claimed in claim 25 wherein said closure is adapted to cover or seal the orifice(s) of the dispenser.

27. A product as claimed in claim 26 wherein said closure is provided with one or more, preferably two or more, e.g. two, protrusions which are complementary in shape to said orifices.

## Patentansprüche

1. Pharmazeutisches und/oder kosmetisches Produkt mit ersten und zweiten einen Wirkstoff enthaltenden Formulierungen zur topischen Verabreichung an einen Patienten, wobei das Produkt eine Aufbewahrungseinrichtung aufweist, wobei die Formulierungen vor der Ausgabe getrennt gehalten werden, zusammen mit einer Ausgabeeinrichtung, die ermöglicht, dass die Formulierungen aus der Aufbewahrungseinrichtung ausgegeben werden, **dadurch gekennzeichnet, dass** (i) ein Wirkstoff in mindestens einer der Formulierungen in einem polymeren Abgabesystem mit porösen Polymerpartikeln enthalten ist, das zur verzögerten Freisetzung des Wirkstoffs in der Lage ist, und (ii) beide Formulierungen Trägerbasen auf Wasserbasis aufweisen, bei denen der Verteilungskoeffizient des Wirkstoffs zwischen den Polymerpartikeln und jeder der Trägerbasen um nicht mehr als 10 % variiert, wobei die erste und die zweite Formulierung Viskositäten aufweisen, die um nicht mehr als 10 % variieren.

2. Produkt nach Anspruch 1, wobei die Formulierungen jeweils wässerige Trägerbasen aufweisen.

3. Produkt nach Anspruch 1 oder Anspruch 2, wobei ein Wirkstoff in mindestens einer der Formulierungen innerhalb eines Microsponge®-Abgabesystems enthalten ist.

4. Produkt nach einem der Ansprüche 1 bis 3, wobei die Ausgabeeinrichtung derart ist, dass sie die Ausgabe der Formulierungen in steuerbaren relativen Mengen ermöglicht.

5. Produkt nach einem vorangehenden Anspruch, wobei die Aufbewahrungseinrichtung nebeneinander liegende Kammern aufweist, die jeweils mit einem Ausgabeventil ausgestattet sind, wobei die Ventile durch benachbart angeordnete Aktuatoren betätigbar sind.

6. Produkt nach einem der Ansprüche 1 bis 4, wobei die Aufbewahrungseinrichtung einen Einheitsdosisbeutel mit separaten Teilen für jede Formulierung aufweist.

7. Produkt nach einem der vorangehenden Ansprüche, wobei die Ausgabeeinrichtung dazu ausgelegt ist, die Formulierungen separat auszugeben.

8. Produkt nach einem der vorangehenden Ansprüche, wobei die erste Formulierung eine wässerige topische Creme- oder Gelträgerbasis ist, die ein antibakterielles und/oder keratolytisches Mittel enthält, das in ein polymeres (z. B. Microsponge) Abgabesystem eingearbeitet ist, das poröse Polymerpartikel aufweist, und die zweite Formulierung eine wässerige Trägerbasis ist, die ein topisches Antibiotikum enthält.

9. Produkt nach Anspruch 8, wobei das keratolytische Mittel ein Retinoid ist.

10. Produkt nach Anspruch 9, wobei das Retinoid Retinsäure ist.

11. Produkt nach Anspruch 8, wobei das antibakterielle Mittel Salicylsäure oder ein organisches Peroxid ist.

12. Produkt nach Anspruch 11, wobei das organische Peroxid Benzoylperoxid ist.

13. Produkt nach einem der Ansprüche 8 bis 12, wobei das Antibiotikum Erythromycin, Clindamycin oder ein Tetracyclin ist.

14. Produkt nach Anspruch 13, wobei das antibakterielle Mittel Benzoylperoxid ist und das Antibiotikum Clindamycin ist.

15. Produkt nach einem der Ansprüche 8 bis 14, wobei das topische Antibiotikum in einem polymeren (z. B. Microsponge) Abgabesystem enthalten ist, das poröse Polymerpartikel aufweist.

16. Produkt nach einem der Ansprüche 1 bis 7, das als Wirkstoffe ein Antipilzmittel und ein Retinoid enthält, von denen mindestens eines in ein polymeres (z. B. Microsponge) Abgabesystem eingearbeitet ist, das poröse Polymerpartikel aufweist.

17. Produkt nach Anspruch 16, wobei das Antipilzmittel Undecylensäure, Miconazol (Base oder Nitrat), Ketoconazol, Iconazol, Clotrimazol ist und das Retinoid entweder Retinol oder Retinsäure ist.

18. Produkt nach einem der Ansprüche 1 bis 7, das als Wirkstoffe ein Depigmentierungsmittel (z. B. Hydrochinon) und ein keratolytisches Mittel (z. B. Salicylsäure) enthält, von denen mindestens eines in ein polymeres (z. B. Microsponge) Abgabesystem eingearbeitet ist, das poröse Polymerpartikel aufweist.

19. Produkt nach einem der Ansprüche 1 bis 7, das als Wirkstoffe ein Depigmentierungsmittel und ein Retinoid enthält, von denen mindestens eines in ein polymeres (z. B. Microsponge) Abgabesystem eingearbeitet ist, das polymere Polymerpartikel aufweist.

20. Produkt nach Anspruch 19, das außerdem ein Corticosteroid, z. B. Hydrocortison, aufweist.

21. Produkt nach einem der Ansprüche 1 bis 7, das als Wirkstoffe ein Corticosteroid (z. B. Hydrocortison) und ein Retinoid (z. B. Retinol) enthält, von denen mindestens eines in ein polymeres (z. B. Microsponge) Abgabesystem eingearbeitet ist, das poröse Polymerpartikel aufweist.

22. Produkt nach einem der Ansprüche 1 bis 7, das als Wirkstoffe einen Haarwuchsförderer (z. B. Minoxidil) und ein Retinoid (z. B. Retinol) oder ein anderes keratolytisches Mittel enthält, von denen mindestens eines in ein polymeres (z. B. Microsponge) Abgabesystem eingearbeitet ist, das poröse Polymerpartikel aufweist.

23. Produkt nach einem der vorangehenden Ansprüche, wobei die erste und die zweite Formulierung im Wesentlichen denselben Wassergehalt aufweisen.

24. Produkt nach einem vorangehenden Anspruch mit mehr als zwei einen Wirkstoff enthaltenden Formulierungen (erste Formulierung, zweite Formulierung, dritte Formulierung usw.) zur topischen Verabreichung an einen Patienten, zusammen mit einer Aufbewahrungseinrichtung, durch die jede der Formulierungen vor der Ausgabe getrennt gehalten wird, wobei (i) ein Wirkstoff in mindestens einer der Formulierungen in einem polymeren Abgabesystem mit porösen Polymerpartikeln enthalten ist, das zur verzögerten Freisetzung des Wirkstoffs in der Lage ist, und (ii) alle Formulierungen Trägerbasen auf Wasserbasis aufweisen, bei denen der Verteilungskoeffizient des Wirkstoffs zwischen den Polymerpartikeln und jeder der Trägerbasen um nicht mehr als 10 % variiert, wobei alle Formulierungen Viskositäten aufweisen, die um nicht mehr als 10 % variieren.

25. Produkt nach einem vorangehenden Anspruch, das ein Doppel- oder Mehrkammer-Ausgabesystem aufweist, das mit einem Aktuator versehen ist, der die Ausgabe der Formulierungen aus einer oder mehreren (vorzugsweise mehreren, z. B. 2) Öffnungen bewirkt, **dadurch gekennzeichnet, dass** das System außerdem mit einem Verschluss versehen ist, der das Herabdrücken des Aktuators verhindert.

26. Produkt nach Anspruch 25, wobei der Verschluss dazu ausgelegt ist, die Öffnung(en) der Ausgabevorrichtung abzudecken oder abzudichten.

27. Produkt nach Anspruch 26, wobei der Verschluss mit einem oder mehr, vorzugsweise zwei oder mehr, z. B. zwei Vorsprüngen versehen ist, die zu den Öffnungen in der Form komplementär sind.

## Revendications

1. Produit pharmaceutique et/ou cosmétique comportant des première et seconde formulations contenant des ingrédients actifs en vue d'une administration topique à un patient, dans lequel ledit produit inclut des moyens de stockage de sorte que lesdites formulations sont maintenues séparément avant distribution, en association avec des moyens de distribution qui permettent de distribuer lesdites formulations à partir desdits moyens de stockage, **caractérisé en ce que** (i) un ingrédient actif dans au moins une desdites formulations est contenu à l'intérieur d'un système de délivrance polymérique comportant des particules de polymère poreuses capables d'une libération retardée dudit ingrédient actif et (ii) lesdites deux formulations comportent des bases de support à base d'eau dans lesquelles le coefficient de partage dudit ingrédient actif entre lesdites particules de polymère et chacune des bases de support ne varie pas de plus de 10 %, dans lequel lesdites première et seconde formulations ont des viscosités qui ne varient pas de plus 10 %.

2. Produit tel que revendiqué dans la revendication 1, dans lequel lesdites formulations comportent chacune des bases de support aqueuses.

3. Produit tel que revendiqué dans la revendication 1 ou 2, dans lequel un ingrédient actif dans au moins une desdites formulations est contenu à l'intérieur d'un système de délivrance Microsponge®.

4. Produit tel que revendiqué dans l'une quelconque des revendications 1 à 3, dans lequel les moyens de distribution sont tels qu'ils permettent une distribution desdites formulations dans des quantités relatives pouvant être commandées.

5. Produit tel que revendiqué dans l'une quelconque des revendications précédentes, dans lequel les moyens de stockage comportent des chambres côte à côte chacune équipées d'une soupape de distribution, lesdites soupapes pouvant être actionnées par des actionneurs disposés de manière adjacente.

6. Produit tel que revendiqué dans l'une quelconque des revendications 1 à 4, dans lequel les moyens de stockage comportent une poche de dose unitaire ayant des parties séparées pour chaque formulation.

7. Produit tel que revendiqué dans l'une quelconque des revendications précédentes, dans lequel les moyens de distribution sont adaptés pour distribuer lesdites formulations de manière séparée.

8. Produit tel que revendiqué dans l'une quelconque des revendications précédentes, dans lequel la première formulation est une crème topique aqueuse ou une base de support en gel contenant un agent antibactérien et/ou kératolytique incorporée dans un système de délivrance polymérique (Microsponge, par exemple) qui comporte des particules de polymère poreuses, et la seconde formulation est une base de support aqueuse contenant un antibiotique topique.

9. Produit tel que revendiqué dans la revendication 8, dans lequel ledit agent kératolytique est un rétinoïde.

10. Produit tel que revendiqué dans la revendication 9, dans lequel ledit rétinoïde est l'acide rétinoïque.

11. Produit tel que revendiqué dans la revendication 8, dans lequel ledit agent antibactérien est l'acide salicylique ou un peroxyde organique.

12. Produit tel que revendiqué dans la revendication 11, dans lequel ledit peroxyde organique est le peroxyde de benzoyle.

13. Produit tel que revendiqué dans l'une quelconque des revendications 8 à 12, dans lequel ledit antibiotique est l'érythromycine, la clindamycine ou une tétracycline.

14. Produit tel que revendiqué dans la revendication 13, dans lequel ledit agent antibactérien est le peroxyde de benzoyle et ledit antibiotique est la clindamycine.

15. Produit tel que revendiqué dans l'une quelconque des revendications 8 à 14, dans lequel ledit antibiotique topique est contenu à l'intérieur d'un système de délivrance polymérique (Microsponge, par exemple) qui comporte des particules de polymère poreuses.

16. Produit tel que revendiqué dans l'une quelconque des revendications 1 à 7, lequel comporte, en tant qu'ingrédients actifs, un agent antifongique et un rétinoïde, dont au moins un est incorporé dans un système de délivrance polymérique (Microsponge, par exemple) qui comporte des particules de polymère poreuses.

17. Produit tel que revendiqué dans la revendication 16, dans lequel l'agent antifongique est l'acide undécilénique, le miconazole (base ou nitrate), le kétoconazole, l'éconazole, le clotrimazole, et le rétinoïde est soit le rétinol soit l'acide rétinoïque.

18. Produit tel que revendiqué dans l'une quelconque des revendications 1 à 7, lequel comporte, en tant qu'ingrédients actifs, un agent de dépigmentation (l'hydroquinone, par exemple) et un agent kératolytique (l'acide salicylique, par exemple), dont au moins un est incorporé dans un système de délivrance polymérique (Microsponge, par exemple) qui comporte des particules de polymère poreuses.

19. Produit tel que revendiqué dans l'une quelconque des revendications 1 à 7, lequel comporte, en tant qu'ingrédients actifs, un agent de dépigmentation et un rétinoïde, dont au moins un est incorporé dans un système de délivrance polymérique (Microsponge, par exemple) qui comporte des particules de polymère poreuses.

20. Produit tel que revendiqué dans la revendication 19, lequel comporte en outre un corticostéroïde (hydrocortisone par exemple).

21. Produit tel que revendiqué dans l'une quelconque des revendications 1 à 7, lequel comporte, en tant qu'ingrédients actifs, un corticostéroïde (hydrocortisone par exemple) et un rétinoïde (rétinol, par exemple), dont au moins un est incorporé dans le système de délivrance polymérique (Microsponge, par exemple) qui comporte des particules de polymère poreuses.

22. Produit tel que revendiqué dans l'une quelconque des revendications 1 à 7, lequel comporte, en tant qu'ingrédients actifs, un stimulant de croissance capillaire (minoxidil, par exemple) et un rétinoïde (rétinol, par exemple) ou un autre agent kératolytique, dont au moins un est incorporé dans un système de délivrance polymérique (Microsponge, par exemple) qui comporte des particules de polymère poreuses.

23. Produit tel que revendiqué dans l'une quelconque des revendications précédentes, dans lequel lesdites première et seconde formulations ont sensiblement la même teneur en eau.

24. Produit tel que revendiqué dans l'une quelconque des revendications précédentes, comportant plus de deux formulations contenant des ingrédients actifs (première formulation, deuxième formulation, troisième formulation, etc.) en vue d'une administration topique à un patient, en association avec des moyens de stockage de sorte que chacune desdites formulations est séparément maintenue avant distribution, dans lequel (i) un ingrédient actif dans au moins une desdites formulations est contenu à l'intérieur d'un système de délivrance polymérique comportant des particules de polymère poreuses capables d'une libération retardée dudit ingrédient actif et (ii) la totalité desdites formulations comportent des bases de support à base d'eau dans lesquelles le coefficient de partage dudit ingrédient actif entre lesdites particules de polymère et chacune des bases de support ne varie pas de plus de 10 %, dans lequel la totalité desdites formulations ont des viscosités qui ne varient pas de plus de 10 %.

25. Produit tel que revendiqué dans l'une quelconque des revendications précédentes, lequel comporte un système de distribution à chambre double ou multiple muni d'un actionneur qui entraîne une distribution desdites formulations à partir d'un ou plusieurs orifices (de préférence une pluralité, par exemple 2), **caractérisé en ce que** ledit système est également muni d'une fermeture qui empêche une dépression dudit actionneur.

26. Produit tel que revendiqué dans la revendication 25, dans lequel ladite fermeture est adaptée pour recouvrir ou sceller l'orifice (ou les orifices) du distributeur.

27. Produit tel que revendiqué dans la revendication 26, dans lequel ladite fermeture est munie d'une ou de plusieurs, de préférence deux ou plus, par exemple deux, saillies qui ont une forme complémentaire de celle desdits orifices.
